# EUROPEAN PATENT APPLICATION

(11) **EP 4 085 972 A1**
(43) Date of publication of application: **09.11.2022**
(21) Application number: 21382415.4
(22) Date of filing: 07.05.2021
(51) Int. Cl.: A61P 25/00

(54) **AMP-ACTIVATED PROTEIN KINASE INHIBITORS FOR USE IN THE TREATMENT OF PTSD IN A SUBJECT**

(71) Applicant: Consejo Superior de Investigaciones Científicas (CSIC), 28006 Madrid (ES); Achucarro Basque Center for Neuroscience Fundazioa, 48940 Leioa (Bizkaia) (ES)
(72) Inventor: FERNÁNDEZ DE SEVILLA GARCÍA-AGENJO, Estrella, Madrid (Madrid) (ES); TORRES ALEMÁN, Ignacio, Leioa (Bizkaia) (ES); PIGNATELLI GARRIGÓS, Jaime, Madrid (Madrid) (ES)
(74) Representative: Pons

(57) **Abstract**

The present invention relates to an AMP-activated protein kinase (AMPK) inhibitor or a pharmaceutical composition comprising said activator in a therapeutically effective amount for use in the treatment and/or prevention of post-traumatic stress disorder (PTSD) in a subject. It also relates to biomarkers of stress susceptibility and their use in a method to determine vulnerability to stress in a subject.

SEQUENCE LISTING

<110> CONSEJO SUPERIOR DE INVESTIGACIONES CIENTÍFICAS (CSIC)
      Achucarro Basque Center for Neuroscience Fundazioa

<120> AMP-ACTIVATED PROTEIN KINASE INHIBITORS FOR USE IN THE TREATMENT OF
PTSD IN A SUBJECT

<130> EP1641.1651

<160> 4

<170> BiSSAP 1.3.6

<210> 1
<211> 24
<212> DNA
<213> Mus musculus

<400> 1
ggttcgttca ctcatggaaa atag                                              24

<210> 2
<211> 24
<212> DNA
<213> Mus musculus

<400> 2
ggtatctctg accagagtca tcct                                              24

<210> 3
<211> 22
<212> DNA
<213> Mus musculus

<400> 3
cttcccagct tgctactcta gg                                                22

<210> 4
<211> 22
<212> DNA
<213> Mus musculus

<400> 4
caggcttgca atgagacatg gg                                                22

## Description

### FIELD OF THE INVENTION

The present invention relates to an AMP-activated protein kinase (AMPK) inhibitor or a pharmaceutical composition comprising said inhibitor in a therapeutically effective amount for use in the treatment and/or prevention of post-traumatic stress disorder (PTSD) in a subject.

### BACKGROUND ART

PTSD is a condition triggered by a terrifying event. Symptoms include flashbacks, nightmares and severe anxiety, as well as uncontrollable thoughts about the event.

Several neuroanatomical studies have identified changes in major brain structures of those with PTSD - the amygdala and hippocampus - showing that there are significant physical changes within the brain as a result of trauma. For example, the prefontal lobe (responsible for language) may be adversely affected by trauma, and its linguistic function be disrupted. Additionally, the amygdala (responsible for emotional regulation) may be in overdrive, due to an increase in its physical size. In contrast, the size of the hippocampus (responsible for memory and experience assimilation) usually shrinks in subjects suffering from PTSD, causing short term memory loss.

Despite PTSD being a relatively common condition, its treatment remains largely unsatisfactory. Understanding how PTSD alters brain chemistry and the knowledge of the mechanisms of vulnerability and resilience to stress disorders is critical to devising treatment and even prevention methods.

### DETAILED DESCRIPTION OF THE INVENTION

Resilience and vulnerability to mood disturbances rely on coping strategies that purportedly show consistent behavioral and neuroendocrine patterns. In this regard, it is known that exposure to a traumatic event, affecting a large part of the world's population, will only trigger PTSD in a relatively small proportion of individuals, probably because of maladaptive coping. Further, specific subsets of the population are at greater risk, such as those exposed to conflicts, because of their greater exposure to trauma. Unfortunately, treatment of PTSD remains largely unsatisfactory. Therefore, knowledge of the mechanisms of vulnerability and resilience to stress disorders are crucial to develop treatments, and even prevent, these illnesses.

The present inventors have recently found that the neuroactive hormone Insuline-like Growth Factor I (IGF-I) is associated with vulnerability to stress in mice and humans. Whilst it is yet to be determined whether the hormone is a neuroendocrine modulator of coping behaviors, regulatory actions of IGF-I on mood are increasingly recognized, pointing to a role in coping strategies. The relation of circulating IGF-I with mood encompasses multiple angles, such as anxiolysis, arousal, playfulness or depression. The latter is profusely documented in the literature, attesting for the increasing recognition of the relevance of IGF-I in mood disorders.

The inventors have focused their search for possible cellular targets of IGF-I in mood modulation on the hypothalamus, a brain area that expresses abundant IGF-I receptors, is involved in fear memories and is central in neuroendocrine regulation of stress. Within the hypothalamus, orexin neurons expressing IGF-I receptors are involved in responses to stress and fear; notably, pharmacological modulation of the activity of said neurons has been proposed as possible therapy for PTSD.

Orexin neurons are also involved in mood regulation in general and in mood-related traits such as motivated behaviors or arousal. Recently, orexin neurons have been shown to be directly involved in PTSD-like responses in rats.

The inventors demonstrate herein the role of orexin neurons in coping behaviors modulated by IGF-I. Surprisingly, IGF-I enables coping behavior through the modulation of the activity of orexin neurons. Additionally, inhibition of AMPK by IGF-I modulates excitatory/inhibitory (E/I) balance onto orexin neurons that participate in behavioral responses to fear. Accordingly, inappropriate systemic IGF-I input onto orexin neurons may interfere with balanced coping behaviors, providing novel targets for therapy of stress disorders.

Thus, the present invention relates to an AMPK inhibitor or a pharmaceutical composition comprising said inhibitor in a therapeutically effective amount for use in the treatment and/or prevention of post-traumatic stress disorder (PTSD) in a subject.

AMPK is a heterotrimeric complex composed of a catalytic α-subunit and two regulatory subunits, β and γ. In humans, AMPK consists of two α-subunits, α1 and α2, encoded by the genes *PRKAA1* (UniProt Q13131, entry 223) and *PRKAA2* (UniProt P54646, entry 208), two β-subunits, β1 and β2, encoded by *PRKAB1* (UniProt Q9Y478, entry 199) and *PRKAB2* (UniProt 043741, entry 193), and three γ-subunits, γ1, γ2 and γ3, encoded by *PRKAG1* (UniProt P54619, entry 192), *PRKAG2* (UniProt Q9UGJ0, entry 183) and *PRKAG3* (UniProt Q9UGI9, entry 161). Each AMPK complex is composed of one α-subunit, one β-subunit and one γ-subunit, and all combinations are possible, thus potentially creating 12 distinct AMPK complexes. Whether these different combinations really represent functionally distinct complexes with different substrate specificities or subcellular localization is yet to be determined.

AMPK has been identified in the past few years as a central integrator of mitochondrial homeostasis by controlling various aspects of the mitochondrial life cycle, from biogenesis and dynamics to removal by mitophagy. AMPK is activated in eukaryotes under conditions in which mitochondrial function and mitochondrial production of ATP are compromised.

Upon changes in energy availability AMPK is activated by an allosteric mechanism that stimulates its kinase activity. Once activated, AMPK redirects metabolism towards increased catabolism and decreased anabolism. In addition to directly regulating key enzymes involved in these pathways, AMPK also rewires cellular metabolism in a prolonged manner by targeting transcriptional regulators.

Almost every mitochondrial insult or defect activates AMPK, including respiratory chain complexes inhibitors such as the diabetes drug metformin, the Parkinson disease-causing herbicide rotenone, proton ionophores, ATP synthase inhibitors and mitochondrial DNA depletion or mutation. Recent findings also implicate mitochondrial dynamics and mitochondrial health in immune cell function and stem cell pluripotency.

Identification of further members of the repertoire of known and confirmed substrates of AMPK at mitochondria would help unveal novel aspects of mitochondrial function regulated by AMPK, and their potential therapeutic role. For example, recent efforts have aimed at generating direct activators of AMPK for the treatment of cancer and metabolic diseases, type 2 diabetes, and general health and lifespan improvement.

The present invention comprises the use of an AMPK inhibitor (hereinafter "inhibitor of the invention"). The term "AMPK inhibitor" refers to any molecule capable of completely or partially inhibiting the biological activity of AMPK by any mode of action, including but not limited to preventing the expression product of the AMPK gene from being produced (interrupting the AMPK gene transcription and/or blocking the translation of the mRNA coming from the AMPK gene expression) and directly inhibiting the AMPK biological activity, for example, and among others, by binding to the enzyme. Methods for decreasing/abrogating the expression of the gene encoding the AMPK protein include, without being limited to, editing technologies such as CRISPR/cas9 or Cas9 nickase technology.

AMPK inhibitors may be identified using methods well known to the skilled person, including but not limited to kinase activity assays or western blotting.

Any AMPK inhibitor may be used in the use of the present invention. However, in certain embodiments of the use of the invention, the AMPK inhibitor may be selected from the group consisting of: (i) IGF; (ii) a small molecule; and (iii) an inhibitor of AMPK gene expression.

In some embodiments of the use of the invention, the AMPK inhibitor is IGF-I (UniProtKB Q13429; version 152).

IGF-I belongs to an ancient family of hormones already present in early invertebrates. It has been considered a peptide mostly involved in the control of body growth and tissue remodeling, with a critical role in the regulation of lifespan and cancer.

In the brain, IGF-I has been traditionally considered a neurotrophic factor involved in brain growth. However, it has recently been suggested that it has a prominent role in brain function as a whole. Indeed, IGF-I has been decribed to modulate brain activity, including higher functions such as cognition.

Different research studies have aimed at determining the role of IGF-1 in neuropsychiatric disorders such as autism, anxiety, depression, PTSD and Alzheimer's disease. However, its role is not completely understood. The present invention characterizes the role of IGF-I in coping behaviour and behavioral responses to fear and stress, demonstrating its criticalk function in the modulation of orexin neurons via inhibition of AMPK.

In some embodiments of the invention, the the AMPK inhibitor is a functional mimetic of IGF-I. In the context of the present invention, a "functional mimetic" or any variations thereof is any kind of biological or chemical molecular entity that activates or represses the same metabolic pathways as IGF-I.

In some embodiments of the invention, the AMPK inhibitor of the invention is a small molecule. Small molecules according to the invention are molecules, typically with a molecular weight less than about 1000 Daltons, or in some embodiments, less than about 500 Daltons, wherein the molecule is capable of modulating, to some measurable extent, an activity of a target molecule, such as AMPK.

In some embodiments of the invention, the small molecule is a functional mimetic of IGF-I.

The inhibitor of the invention may also be an inhibitor of the AMPK gene expression. Said inhibitor may be, among others, siRNA, an antisense oligonucleotide, a nuclease or a ribozyme.

Small interference RNA or siRNA are agents which are capable of inhibiting the expression of a target gene by means of RNA interference. A siRNA can be chemically synthesized, can be obtained by means of *in vitro* transcription or can be synthesized *in vivo* in the target cell. Typically, the siRNA consists of a double stranded RNA between 15 and 40 nucleotide long and may contain a 3' and/or 5' protruding region of 1 to 6 nucleotides. The length of the protruding region is independent of the total length of the siRNA molecule. The siRNA acts by means of degrading or silencing the target messenger after transcription.

The siRNA of the invention is substantially homologous to the mRNA of the AMPK encoding gene or to the gene sequence which encodes said protein. "Substantially homologous" is understood as having a sequence which is sufficiently complementary or similar to the target mRNA such that the siRNA is capable of degrading the latter through RNA interference. The siRNA suitable for causing said interference include siRNA formed by RNA, as well as siRNA containing different chemical modifications such as: (i) siRNA in which the bonds between the nucleotides are different than those appear in nature, such as phosphorothionate bonds; (ii) Conjugates of the RNA strand with a functional reagent, such as a fluorophore; (iii) Modifications of the ends of the RNA strands, particularly of the 3' end by means of the modification with different hydroxyl functional groups in 2' position; (iv) Nucleotides with modified sugars such as 0-alkylated residues on 2' position like 2'-O-methylribose or 2'-O-fluororibose; or (v) Nucleotides with modified bases such as halogenated bases (for example 5-bromouracil and 5-iodouracil), alkylated bases (for example 7-methylguanosine).

The siRNA can be used in the form of a double stranded RNA with the aforementioned characteristics. Alternatively, the use of vectors containing the sense and antisense strand sequence of the siRNA is possible under the control of suitable promoters for the expression thereof in the cell of interest.

Vectors suitable for expressing siRNA are those in which the two DNA regions encoding the two strands of siRNA are arranged in tandem in one and the same DNA strand separated by a spacer region which, upon transcription, forms a loop and wherein a single promoter directs the transcription of the DNA molecule giving rise to shRNA.

The siRNA can be generated intracellularly from the so-called shRNA (short hairpin RNA) characterized in that the antiparallel strands forming the siRNA are connected by a loop or hairpin region. The shRNAs can be encoded by plasmids or viruses, particularly retroviruses, and are under the control of a promoter. Promoters suitable for expressing shRNA are those indicated in the paragraph above for expressing siRNA.

The siRNA and shRNA of the invention can be obtained using a series of techniques known by the person skilled in the art.

Isolated "antisense" nucleic acids may also be used to inhibit AMPK expression, for example, for inhibiting transcription and/or translation of a nucleic acid which encodes AMPK, the activity of which is to be inhibited. The antisense nucleic acids can be bound to the target potential of the drug by means of conventional base complementarity or, for example, in the case of binding to double stranded DNA through specific interaction in the large groove of the double helix. Generally, these methods refer to a range of techniques generally used in the art and they include any method which is based on the specific binding to oligonucleotide sequences.

An antisense oligonucleotide can be distributed, for example, as an expression plasmid which, when it is transcribed in cell, produces RNA complementary to at least one unique part of the cellular mRNA encoding AMPK. Alternatively, the antisense oligonucleotide is an oligonucleotide probe generated *ex vivo* which, when introduced into the cell, produces inhibition of gene expression hybridizing with the mRNA and/or gene sequences of a target nucleic acid. Such oligonucleotide probes are preferably modified oligonucleotides which are resistant to endogenous nucleases, for example, exonucleases and/or endonucleases and are therefore stable in vivo. Examples of nucleic acid molecules for use thereof as antisense oligonucleotides are DNA analogs of phosphoramidate, phosphothionate and methyl phosphonate.

With respect to the antisense oligonucleotide, the oligodeoxyribonucleotide regions derived from the starting site of the translation, for example, between -10 and +10 of the target gene are preferred. The antisense approximations involve the oligonucleotide design (either DNA or RNA) that are complementary to the mRNA encoding the target polypeptide. The antisense oligonucleotide will be bound to the transcribed mRNA and translation will be prevented.

The oligonucleotides which are complementary to the 5' end of the mRNA, for example the non-translated 5' sequence up to and including the start codon AUG must function in the most efficient manner to inhibit translation. Nevertheless, it has been shown that the sequences complementary to the non-translated 3' sequences of the mRNA are also efficient for inhibiting mRNA translation. Therefore, complementary oligonucleotides could be used at the non-translated 5' or 3' regions, non-coding regions of a gene in an antisense approximation to inhibit the translation of that mRNA. The oligonucleotides complementary to the non-translated 5' region of the mRNA must include the complement of the start codon AUG. The oligonucleotides complementary to the coding region of the mRNA are less efficient translation inhibitors but they could also be used according to the invention. If they are designed to hybridize with the 5' region, 3' region or the coding region of the mRNA, the antisense nucleic acids must have at least six nucleotides long and preferably have less than approximately 100 and more preferably less than approximately 50, 25, 17 or 10 nucleotides long.

Preferably, *in vitro* studies are performed first to quantify the capacity of the antisense oligonucleotides for inhibiting gene expression. Preferably these studies use controls which distinguish between antisense gene inhibition and non-specific biological effects of the oligonucleotides. Also, preferably these studies compare the levels of target RNA or protein with that of an internal control of RNA or protein. The results obtained using the antisense oligonucleotides can be compared with those obtained using a control oligonucleotide. Preferably the control oligonucleotide is approximately of the same length as the oligonucleotide to be assayed and the oligonucleotide sequence does not differ from the antisense sequence more than it is deemed necessary to prevent the specific hybridization to the target sequence.

The antisense oligonucleotide can be a single or double stranded DNA or RNA or chimeric mixtures or derivatives or modified versions thereof. The oligonucleotide can be modified in the base group, the sugar group or the phosphate backbone, for example, to improve the stability of the molecule, its hybridization capacity, etc. The oligonucleotide may include other bound groups, such as peptides (for example, for directing them to the receptors of the host cells), agents for facilitating transport through the cell membrane or the blood-brain barrier, and intercalating agents. For this purpose, the oligonucleotide can be conjugated to another molecule, for example, a peptide, a transporting agent, hybridization triggered cleaving agent, etc.

The antisense oligonucleotides may comprise at least one group of modified bases. The antisense oligonucleotide may also comprise at least a modified sugar group selected from the group including but not limited to arabinose, 2-fluoroarabinose, xylulose, and hexose. The antisense oligonucleotide may also contain a backbone similar to a neutral peptide. Such molecules are known as peptide nucleic acid (PNA) oligomers. In yet another embodiment, the antisense oligonucleotide comprises at least one modified phosphate backbone. In yet another embodiment, the antisense oligonucleotide is an alpha-anomeric oligonucleotide.

While antisense oligonucleotides complementary to the coding region of the target mRNA sequence can be used, those complementary to the transcribed non translated region can also be used.

In some cases, it may be difficult to reach the sufficient intracellular concentrations of the antisense to suppress the endogenous mRNA translation. Therefore, a preferred approximation uses a recombinant DNA construct in which the antisense oligonucleotide is placed under the control of a strong pol III or pol II promoter.

Alternatively, the target gene expression can be reduced by directing deoxyribonucleotide sequences complementary to the gene regulating region (i.e., the promoter and/or enhancers) to form triple helix structures preventing gene transcription in the target cells in the body. In certain embodiments, the antisense oligonucleotides are antisense morpholines.

The present invention also contemplates the use of DNA enzymes to inhibit the expression of the AMPK gene. DNA enzymes incorporate some of the mechanistic features of both antisense and ribozyme technologies. DNA enzymes are designed such that they recognize a particular target nucleic acid sequence similar to the antisense oligonucleotide, nevertheless like the ribozyme they are catalytic and specifically cleave the target nucleic acid.

Additionally, the present invention also contemplates the use of ribozymes to inhibit the expression of the AMPK gene. Ribozyme molecules designed for catalytically cleaving transcription products of a target mRNA to prevent the translation of the mRNA which encodes AMPK, the activity of which is to be inhibited, can also be used. Ribozymes are enzymatic RNA molecules capable of catalyzing specific RNA cleaving. The mechanism of ribozyme action involves a specific hybridization of a ribozyme molecule sequence to a complementary target RNA followed by an endonucleolytic cleavage event. The composition of the ribozyme molecules preferably includes one or more sequences complementary to the target mRNA and the well-known sequence responsible for cleaving the mRNA or a functionally equivalent sequence. Examples of ribozymes to be used in the present invention include hammer-head ribozymes and endoribonuclease RNA (hereinafter "Cech type ribozymes").

The ribozymes can be formed by modified oligonucleotides (for example to improve the stability, targeting, etc.) and they should be distributed to cells expressing the target gene *in vivo.* A preferred distribution method involves using a DNA construct which "encodes" the ribozyme under the control of a strong constitutive pol III or pol II promoter such that the transfected cells will produce sufficient amounts of the ribozyme to destroy the endogenous target messengers and to inhibit translation. Since the ribozymes are catalytic, unlike other antisense molecules, a low intracellular concentration is required for its efficiency.

The present invention relates to an AMPK inhibitor for use in the treatment and/or prevention of PTSD.

As used herein, the term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above).

As used herein, the term "preventing" (or "prevent" or "prevention") refers to the capacity of the AMPK inhibitor to minimize or hinder the progression of a disease, condition or disorder in a subject.

As used herein, the term "subject" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In a preferred embodiment, the subject is a human, man or woman of any age or race.

In the present invention, the condition or disorder to be treated or prevented is PTSD.

PTSD is a condition that may develop after exposure to exceptionally threatening or horrifying events. It can occur after a single traumatic event or from prolonged exposure to trauma. However, predicting who will go on to develop PTSD is a challenge; many people show remarkable resilience and capacity to recover following exposure to trauma.

The development of PTSD may be facilitated by a failure to contain the biologic stress response at the time of the trauma, resulting in a cascade of alterations that lead to PTSD. The biologic alterations observed in PTSD do not uniformly resemble those associated with other types of stress. For example, cortisol levels have been lower than normal in some studies of patients with PTSD, whereas corticotropin-releasing factor in cerebrospinal fluid appear to be increased. This pattern differs from the patterns associated with brief and sustained periods of stress and with major depression, which are typically associated with increased levels of both cortisol and corticotropin-releasing factor. Furthermore, it has been shown that patients with chronic PTSD have even more increased circulating levels of norepinephrine and reactivity of adrenergic receptors. These alterations, in addition with the findings that thyroid hormone levels are generally increased in patients with PTSD, also explain some of the somatic or physical symptoms of the PTSD.

Patients with PTSD are at increased risk of experiencing poor physical health, including somatoform, cardiorespiratory, musculoskeletal, gastrointestinal, and immunological disorders. PTSD is also associated with substantial psychiatric comorbidity, increased risk of suicide, and considerable economic burden. However, there is no pharmacological treatment available, with a significant lack of robust evidence supporting the use of drug interventions. Knowledge of the biological mechanisms associated with stress disorders are crucial to develop treatments.

In some embodiments of the invention, the inhibitor of the invention is a small molecule.

In some specific embodiments of the invention, the small molecule is 6-[4-(2-Piperidin-1-yl-ethoxy)-phenyl)]-3-pyridin-4-yl-pyrrazolo[1,5-a]-pyrimidine (Compound C; CAS 866405-64-3).

In a preferred embodiment of the use of the invention, the AMPK inhibitor is comprised within a pharmaceutical composition in a therapeutically effective amount. The term "therapeutically effective amount" means the amount of AMPK inhibitor that will elicit the biological or medical response of a tissue, system, animal or human that is being sought by the researcher, veterinarian, medical doctor or other clinician. In the context of the present invention, the biological or medical response to elicit is the treatment and/or prevention of PTSD.

The AMPK inhibitors of the present invention can be incorporated into pharmaceutical compositions for its administration to the subject. The AMPK inhibitors of the present invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. Oral tablets may also be formulated for immediate release, such as fast melt tablets or wafers, rapid dissolve tablets or fast dissolve films.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

The AMPK inhibitor of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions and the like, containing the compounds of the present invention are employed. Similarly, transdermal patches may also be used for topical administration.

In the treatment, prevention, control, amelioration, or reduction of risk of hematologic malignancies, an appropriate dosage level of the AMPK inhibitor of the invention will generally be about 0.01 to 500 mg per kg patient body weight per day which can be administered in single or multiple doses. For oral administration, the compositions may be provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1,000.0 milligrams of the AMPK inhibitor for the symptomatic adjustment of the dosage to the patient to be treated.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The pharmaceutical composition of the invention may further comprise a compound for the treatment of PTSD in a subject.

In some embodiments of the invention, the AMPK inhibitor or composition of the invention modulates the activity of orexin neuronal circuits in the hypothalamus.

The orexin-A/hypocretin-1 and orexin-B/hypocretin-2 are neuropeptides synthesized by a cluster of neurons in the lateral hypothalamus and perifornical area. Orexin neurons receive a variety of signals related to environmental, physiological and emotional stimuli, and project broadly to the entire CNS. They are "multi-tasking" neurons regulating vital body functions, including sleep/wake states, feeding behavior, energy homeostasis, reward systems, cognition and mood.

A dysfunction of orexinergic system may underlie different pathological conditions. A selective loss of orexin neurons has been found in narcolepsia, supporting the crucial role of orexins in maintaining wakefulness. Interestingly, orexinergic neurons show connections to regions involved in cognition and mood regulation, including hippocampus. Orexins enhance hippocampal neurogenesis and improve spatial learning and memory abilities, and mood. Conversely, orexin deficiency results in learning and memory deficits, and depression.

Orexin neurons expressing IGF-IR are involved in responses to stress and fear; pharmacological modulation of their activity has previously been proposed as possible therapy for PTSD. Electrophysiological studies on transgenic mice have identified several neurotransmitters and neuromodulators influencing the activation or inhibition in orexin neurons activity, specifically GABA, noradrenaline, serotonin and dopamine. As shown by the inventors for the first time herein, IGF-I enables coping behavior through balancing orexin activity. Firoc mice lacking functional IGF-IR receptors in orexin neurons develope PTSD-like behavior after fear conditioning, and show excess orexinergic activity that when inhibited, ameliorates PTSD-like traits. In wild type mice, inhibition of orexin activity or treatment with IGF-I, ameliorates PTSD-like behavior developed after intense fear exposure, unveiling the regulatory role of IGF-I in excitatory/inhibitory inputs onto orexin neurons.

In further inventive aspects, the present invention also encompasses a method of treating or preventing PTSD in a subject comprising the use of the inhibitor or the composition of the invention. It also encompasses the use of the inhibitor or composition of the invention as described herein in the manufacture of a medicament for the treatment or prevention of PTSD in a subject. All the preferred embodiments and explanations of the terms used in these inventive aspects have been explained above.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE FIGURES

**Figure 1****.** Anxiolytic actions of IGF involve orexin neurons.
**Figure 2****.** IGF-I modulates fear learning through orexin neurons.
**Figure 3****.** Firoc mice show abnormal fear learning and PTSD-like traits.
**Figure 4****.** Changes in gene expression in Firoc mice after fear learning.
**Figure 5****.** Orexin neurons participate in development of PTSD-like behavior in wild type mice and IGF-I prevents it.
**Figure 6****.** Firoc mice show unbalanced Glutamatergic/GABAergic input onto orexin neurons.
**Figure 7****.** Fear conditioning reverses Glutamatergic/GABAergic input onto orexin neurons in Firoc mice.
**Figure 8****.** IGF-I modulates GABAergic and Glutamatergic inputs onto orexin neurons.
**Figure 9****.** AMPK is involved in regulation of orexin activity by IGF-I.
**Figure 10****.** Firoc mice present normal escape behaviour and anxiety levels of mice under basal conditions.
**Figure 11****.** Firoc mice do not present anhedonia under basal condicions. Treatment of fear-conditioned Firoc mice treated with IGF-I does not ameliorate the exaggerated freezing, whereas treatment with Compound C does.
**Figure 12****.** Spike firing in orexinergic neurons of Firoc-ChR and Control-ChR.

### EXAMPLES

### Materials and methods

Antibodies used are shown in Table 1. Human recombinant IGF-I was from Pre-Protech (USA). For hdM4Di (DREADD) experiments, clozapine-N-oxide (CNO) from Tocris was administered at 2mg/kg dissolved in saline 0.9%, and injected intraperitoneally 40 min before test sessions, 24h and 1week after fear conditioning.

**Table 1. Antibodies used in this study.**

| **Primary antibody** | **Host** | **Company** |
|---|---|---|
| **Anti-orexin-A** | Mouse | Santa Cruz, sc-80263 |
| **Anti-orexin-A** | Rabbit | Abcam, ab6214 |
| **Anti-c-Fos** | Rabbit | Abcam, ab190289 |
| **Anti-Cre** | Mouse | Millipore, MAB3120 |
| **Anti-tyrosine hydroxilase** | Mouse | Millipore, MAB318 |
| **Anti-VGLUT2** | Guinea Pig | Synaptic Systems, 135 404 |
| **Anti-VGAT** | Mouse | Synaptic Systems, 131 011 |
| **Anti-GABBR2 (phospho S783)** | Rabbit | Abcam, ab72447 |
| **Anti-GluR1 (phospho Ser845)** | Rabbit | Novus Biologicals, NB300-171 |
| **Anti-Phospho-AMPKa (Thr172)** | Rabbit | Cell Signaling, 2535 |
| **Anti- melanocyte concentrating hormone (MCH)** | Rabbit | Abcam, ab274415 |

Animals. Adult female and male C57BL/6J mice (Harlan Laboratories, Spain), and Cre/Lox mice lacking IGF-I receptors in orexin neurons (Floxed IGF-IR/Orexin Cre or Firoc mice) were used. Firoc mice were obtained by crossing Orexin-Cre mice (a kind gift of Dr T Sakurai, Tsukuba Univ, Japan; (Matsuki et al., 2009) with IGF-IRf/f mice (B6, 129 background; Jackson Labs; stock number: 012251) as explained in detail elsewhere (Zegarra-Valdivia et al., 2020). Orexin neurons in Firoc mice do not respond to systemic IGF-I administration, as assessed by c-fos and phospho-Akt expression (Zegarra-Valdivia et al., 2020).

Genotyping of Firoc mice was performed using 5'-GGTTCGTTCACTCATGGAA AATAG-3' (SEQ ID NO: 1), and 5'-GGTATCTCTGACCAGAGTCATCCT-3' (SEQ ID NO: 2) for Orexin-Cre and 5'-CTTCCCAGCTTGCTACTCTAGG-3' (SEQ ID NO: 3), and 5'-CAGGCTTGCAATGAGACATGGG-3' (SEQ ID NO: 4) for IGF-IRf/f. DNA from brain tissue was isolated using Trizol Reagent and ethanol precipitation. 10ng of genomic DNA was used in a PCR reaction containing 1X reaction buffer, 1µM of each primer, 0.2mM of dNTPS and 0.75ul of DFS-Taq DNA polymerase (Bioron, GmbH). The thermocycler program was 92°C, 3 min and 30 cycles of 94°C, 30sec; 65°C, 30sec; 72°C, 30sec, after that a final extension step at 72°C for 2 min was performed. Amplicons were analyzed in 3% agarose gels stained with SYBRsafe (Thermofisher, Inc).

Animals were housed in species-specific standard cages (mice 5 per cage; rats 1-2 rats per cage), and kept in a room with controlled temperature (22°C) under a 12-12h light-dark cycle. All animals were fed with a pellet rodent diet and water ad libitum. All experimental protocols were performed during the light cycle. Mice were handled for 3 days prior to any experimental manipulations. Animal procedures followed European guidelines (86/609/EEC and 2003/65/EC, European Council Directives) and were approved by the local Bioethics Committee (Government of the Community of Madrid).

Viral constructs. For chemogenetic experiments using DREADD, a viral construct (pAAV-hSyn-DIO-hM4D(Gi)-mCherry (AAV5; Addgene # 44362); 8.6 x 1012 viral infective units/ml) was locally injected bilaterally to inactivate orexin-cre neurons in Orexin-Cre (littermates) and transgenic mice (Firoc). As control virus, pAAV-hSyn-DIO-mCherry (AAV5; Addgene # 50459) was used. Both viral constructions were obtained from Addgene. Clozapine N-Oxide (CNO, 2mg/kg dissolved in saline 0.9%) was administered ip and 40 min later behavior was assessed, CNO efficacy in orexin neurons was confirmed in acute slices obtained from injected mice. Slices for electrophysiological recordings were prepared from 2-months old Orexin-Cre mice, 4 weeks after injection of the DREADD-mCherry virus into the lateral hypothalamus. Brains were quickly removed and coronal slices (250 µm) containing the lateral hypothalamus were cut with a vibratome (4°C) in a solution containing: 234 mM sucrose, 11 mM glucose, 26 mM NaHCO3, 2.5 mM KCI, 1.25 mM NaH2PO4, 10 mM MgSO4, and mM 0.5 CaCl2 (equilibrated with 95% O2-5% CO2). Recordings were obtained at 30-32 oC from orexin+ neurons identified using fluorescence microscopy (mCherry+) in oxygenated artificial cerebrospinal fluid containing the following: 126 mM NaCl, 26 mM NaHCO3, 2.5 mM KCI, 1.25 mM NaH2PO4, 2 mM MgSO4, 2 mM CaCl2 and 10 mM glucose (pH 7.4). Patch-clamp electrodes contained intracellular solution composed of: 131 mM K gluconate, 5 mM KCI, 4 mM MgCI2, 10 mM HEPES, 4 mM EGTA, 2 mM MgATP, and 0.3 mM Na2GTP (pH 7.3) corrected with KOH (290 mOsm). Positive and negative currents were injected during 600 ms to calculate action potential frequency and input resistance. CNO (2µM) was applied through perfusate dissolved in ACSF. Signals were amplified, using a Multiclamp 200B patch-clamp amplifier (Axon Instruments, Foster City, California, United States), sampled at 20 kHz, filtered at 10 kHz, and stored on a PC. Data were analyzed using pClamp (Axon Instruments). CNO-mediated inhibition of orexin neurons was confirmed in hypothalamic slices of Orexin-Cre mice transduced with pAAV-hSyn-DIO-hM4D(Gi)-mCherry.

For inactivation of IGF-IR in orexin neurons of adult mice, a pAAV-Orexin-Cre virus (AAV-Firoc) or a AAV-CMV-EGFP virus (AAV-Control; Addgene # 67634) were bilaterally injected in the lateral hypothalamus (stereotaxic coordinates: AP= -1.4; ML= ± 0.9; DV= -5.4) of IGF-IRf/f mice (3 months-old). The mice were submitted to fear-learning one month later (see below). The HCRT minipromoter (Ple112; (de Leeuw et al., 2014) used in this construction was obtained from plasmid pEMS1418 (Addgene # 29214) by enzymatic restriction digestion, and inserted into a pAAV-CMV-CRE-ires-GFP-WPRE plasmid (Addgene # 105545), in a cloning step that implies the substitution of the CMV promoter by the HCRT mini-promoter. The resulting plasmid pAAV-HCRT-CRE-ires-GFP-WPRE was used for AAV particles packaging (Viral Vector Production Unit, UAB-VHIR, Barcelona, Spain.).

Surgery. For all surgeries, mice were anaesthetized with isoflurane (Zoetis) administered with a nose mask (David Kopf Instruments, France), and placed on a stereotaxic frame (Stoelting Co) on a heating pad and tape in their eyes to protect them from light. For viral expression, mice were injected with a 5 µl Hamilton syringe bilaterally into the orexin nuclei (AP= -1.4; ML= ± 0.9; DV= -5.4) 4 weeks before experiments. For DREADD viral expression, 300 nl were bilaterally infused at a rate of 100 nl/min and the Hamilton syringe withdrawn 10 min later.

### Behavioral tests

Open field. Locomotion and exploratory behavior were evaluated placing the animal in an open field arena (42 cm× 42 cm x 30 cm, Versamax; AccuScan Instruments, Inc.) for 10 min. All parameters were quantified automatically with the software provided.

Cued-fear conditioning. Experiments were performed at the same time of the day during the light phase with 3-4 months-old male and female mice (n=14-19 mice/genotype, balanced sex). The training and test sessions were video recorded. Mice were placed in a shuttle box chamber (AccuScan Instruments) for 120 sec before exposing them to a neutral conditioned stimulus (CS+) -a tone of 80 dB for 30s, presented together with an aversive unconditioned stimulus (US) -an electrical footshock of 0.3 mA/2 sec, except in the case of the PTSD-eliciting protocol, where 2.5 mA shocks were used. Training consisted of 5 consecutive trials with 90 sec intertrials (CS-). Animals remained in the chamber for 5 min more before returning them to their home cages. Twenty-four hours later, fear conditioning was tested placing the animals in a different context using the same protocol but without the unconditioned stimulus (US). As a consequence of the US-CS association, mice displayed freezing behavior, which was scored for every trial (CS+), and intertrial (CS-). Freezing behavior was defined by the absence of movement except of breathing and heart beating. Context-dependent extinction was tested 1 and 5 weeks later (delayed extinction). Mice were placed in the same context for 5 min to measure their freezing behavior. The latter unveils PTSD-like behaviour when it is abnormally retained.

Predator exposure test. Control littermates and Firoc mice implanted with osmotic minipumps (either with saline or IGF-I, see below), were introduced into a box for 10 min with two compartments separated by a plastic grid, one containing a rat (predator) and the other empty. The box contained bedding of the rat with urine and feces. All sessions were recorded, and every rat exposed to a maximum of 4 mice/day. Grid contacts, freezing time and bedding burying behaviors of exposed mice were scored.

Elevated plus maze. Two days after exposure to the rat, mice were introduced in a maze of 40 cm from the floor with two opposing protected (closed) arms of 30 cm (length) x 5 cm (wide) x 15.25 (height), and two opposing unprotected (open) arms of 30 cm (length) x 5 cm (wide). Each animal was introduced in the center of the maze for 10 min. Stress was scored as time animals spent in closed arms. All measures were recorded with an automated video-tracking system (Video Tracking Plus Maze Mouse; Med Associates, USA).

Sucrose preference test. Mice were given 2 bottles of water for 3 days, and then 2 bottles of 2% sucrose for 2 days. Afterwards, mice were deprived of food and water for 18h and then, they were presented a bottle of water and a bottle of 2% sucrose during 2h. The position of the bottles was switched after 1h. Bottles were placed in the active phase. Water and sucrose consumption was recorded and sucrose preference was defined as the ratio of the weight of sucrose intake to the weight of total intake of liquid (water and sucrose).

Escape test. Mice were introduced in a shuttle box chamber (AccuScan Instruments) which is separated in two compartments. The test comprises 5 trials, separated by intertrials of 30s. Each trial consisted of an escapable foot shock of 10s/0.1 mA which stops when the animal crosses to the opposite compartment (Lecca et al., 2017). This behavior is learned easily when more trials are performed (Mayeaux et al., 2015). Latencies to escape from the shock were quantified with Versamax software.

Immunocytochemistry. Mice were perfused transcardially under deep anesthesia (sodium pentobarbital, 50 mg/kg, i.p.) with 100 ml of saline buffer 0.9% followed by 100 ml of 4% paraformaldehyde (PFA) in 0.1N, pH 7.4 phosphate buffer (PB), 90 min after the cued-fear conditioning test, the recall context, or under basal conditions. This time point was selected as optimal to see c-Fos staining. For experiments with systemic administration of IGF-I, animals were sacrificed 2 hours after injection. Brains were removed, post-fixed overnight at 4°C in the same fixative, and cut at 50 µm thick sections on a vibratome (Leica VT 1000S). Sections were kept at 4°C, immersed in 0.1N PB with 0.02% sodium azide, until processing. Serial coronal free-floating sections were rinsed in 0.1N PB for 10 min, and a blocking solution containing 10% normal donkey serum, and 0.4% Triton X-100 in 0.1N PB (PB-T) was added and maintained at room temperature for 2 h. Thereafter, sections were incubated overnight at 4°C in the same solution with the corresponding primary antibodies (see Antibody Table). The next day, sections were washed 3 times with PB-T, and incubated with secondary antibodies (see Antibody Table) for 2 h at room temperature. After the incubation, slices were washed 3 times with PB-T and incubated 5 min with Hoechst (1:500 dilution, Life Technologies). Finally, sections were washed 3 times with PB and mounted onto glass slides coated with gelatin in Gelvatol mounting medium. For double-immunostaining of MCH/ pGABA(B)R2, when antibodies were done in the same host, we performed a sequential immunolabeling. Firstly, as previously described, we incubated with anti-MCH antibody and his corresponding secondary antibody. Sections were washed 3 times with PB-T, fixed with 1% PFA for 10 min and blocked again for 1 h. Then, tissue was incubated with primary anti-pGABA(B)R2 antibody overnight and thereafter with its corresponding secondary antibody. Final steps were as above. Images were taken with confocal microscopy (SP5, Leica Microsystems, Germany) and analyzed with Imaris software (Bitplane).

Image analysis. With the images taken at 20x magnification, spots were created with an estimated XY size of 15 µm to count orexin+ or TH+ neurons in their channel (red), and next a filter corresponding to the c-Fos channel (green) was added to calculate the number of orexin+/c-Fos+ and TH+/c-Fos+ cells. Similar procedures were used when evaluating double-labelled (orexin or MCH) neurons with pSer783/893GABAR(B), pSer845GluR1, and pThr172AMPK. To score excitatory and inhibitory synaptic inputs in orexin neurons triple immunocytochemistry was performed with Vglut2 (excitatory) and Vgat (inhibitory) antibodies (see Table 1). As explained elsewhere by others (Fogarty et al., 2013), a surface in the images was created at 63x magnification corresponding to all orexin neuron surfaces in their channel (gray). Then, to filter the Vgat (green) and Vglut2 (red) channels, the channels were masked in the orexin surface, to only maintain that portion of the channels. Last, spots were created for Vgat and Vglut2 with an estimated XY size of 0.5 µm, and the number of Vgat and Vglut2 spots in orexin+ cells was counted.

qPCR. Animals were anesthetized with pentobarbital (50 mg/kg, ip). After dissecting the brain, the different brain areas were collected and frozen at -80°C until use. Tissue RNA was extracted with Trizol (Life Technologies, USA), as described elsewhere (Santi et al., 2018a). cDNA was synthesized from 1µg of RNA of each sample following the manufacturer's instructions (Hight Capacity cDNA Reverse Transcription Kit; Applied Biosystems). Fast Real-time qPCR was performed using the SYBR Green method (Fast SYBR Green Master Mix, Applied Biosystems) with the QuantStudio 3 Real-Time PCR System (Applied Biosystems). Relative mRNA expression was determined by the 2-ΔΔCT method (Pfaffl, 2001), and normalized to GAPDH levels.

Drug administration. Human recombinant IGF-I (Pre-Protech, USA) or vehicle (saline) were administered intracerebroventricularly (icv, 50 µg/kg/day) using Alzet osmotic minipumps for 7 days (Model 1007D), and the brain infusion kit 3 (Alzet, USA) with the following stereotaxic coordinates: AP= -0.5; ML= 1.1; DV= -2.5. Alzet pumps were implanted subcutaneously between the scapulae. In other experiments IGF-I was given intraperitoneally (ip, 1 µg/kg) to wild type mice submitted to the 2.5 mA fear-learning protocol 6 hours after fear conditioning. For hdM4Di (DREADD) experiments, clozapine-N-oxide (CNO, Tocris) was administered at 2mg/kg dissolved in saline 0.9%, and injected intraperitoneally 40 min before test sessions, and 24h and 1week after fear conditioning. 6-[4-(2-Piperidin-1-yl-ethoxy)-phenyl)]-3-pyridin-4-yl-pyrrazolo [1,5-a]-pyrimidine dihydrochloride (Compound C dihydrochloride, AMPK inhibitor; Cat. # CD0339, Chemdea NJ, USA) was administered intraperitoneally (ip, 10 mg/kg) 6 hours after fear conditioning to PTSD-like wild type mice.

Recordings in anesthetized animals. Electrophysiological recordings were performed as described (Zegarra-Valdivia et al., 2020). Briefly, Control and Firoc mice were anesthetized with isoflurane (2% induction; 1-1.5% in oxygen, maintenance doses), placed in a David Kopf stereotaxic apparatus (Tujunga, CA, USA) in which surgical procedures and recordings were performed, with a warming pad (Gaymar T/Pump, USA) set at 37°C. Local anesthetic (lidocaine 1%) was applied to all skin incisions and pressure points. An incision was made exposing the skull, and small holes were drilled in the skull. An optrode (Optical fiber + Tungsten microelectrode of 0.5-0.8 MΩ, with a core diameter 120 µm; Thomas Recording) was used to record the evoked potential in the LH/PeF area (coordinates from Bregma: A, -1.95; L, 1.0 and depth, 4.0 - 4.5mm). Multi-unitary recordings were performed through the Tungsten microelectrode of the optrode. Signal was filtered (0.3-3 kHz) and amplified using a DAM80 preamplifier (World Precision Instruments). The locus coeruleus (LC) was stimulated using 120 µm diameter stainless steel bipolar electrodes (World Precision Instruments, coordinates from Bregma: A, -5.4; L, 1.0 and depth, 4.0 - 4.5mm) with single square pulses (0.3 ms duration and 20-50 µA intensity, delivered at 1 Hz; Cibertec Stimulator, Spain) to elicit potential activation in the LH/PeF area (coordinates from Bregma: A, -0.2; L, 0.8 and depth, 4.5 - 5mm). After a basal recording, a second electric stimulation was performed in the lateral preoptic area (LPO, single square pulses, 0.3 ms duration, and 20-50 µA intensity, delivered at 1 Hz; Cibertec Stimulator, Spain) with a delay of 100 ms before LC stimulation to induce inhibition in the LH/PeF area. The multi-unitary activity in the LH/PeF in basal condition was compared to the multi-unitary activity under LPO stimulation.

Optogenetics. Optogenetic experiments were performed to identify orexin neurons through light activation using mice expressing channelrhodopsin under the orexin promoter (Ox-ChR mice) as controls and Firoc-ChR mice, as described (Zegarra-Valdivia et al., 2020). Animals were anesthetized with isoflurane, positioned in the stereotaxic apparatus, and handled as above. The scalp's sagittal midline was sectioned and retracted, and a small craniotomy was drilled over the Perifornical (LH/PeF) hypothalamic area (same stereotaxic coordinates as above). Optical stimulation of ChR-expressing neurons was achieved with light-emitting diodes (LED; 300 ms pulse; Thomas Recording, Germany). Multi- and single-unit recordings were performed through the optrode, filtered (0.3-3 kHz), and amplified using a DAM80 preamplifier (World Precision Instruments). Multi-unit and single-unit activities were sampled with the aid of Spike2 software (Cambridge Electronic Design, UK) at 10 kHz via an analog-to-digital converter built into the Power 1401 data acquisition unit and fed into a PC for off-line analysis with Spike 2 software. Unit activity was extracted from the recording using a filter from 0.3-3 kHz. Multi-unit and single-unit responses elicited by LC stimulation in the LC/PeF area were calculated using a per-stimulus time histogram (1 ms bin). A square-step voltage command triggered the LED. A single long-lasting pulse was applied (473 nm light, 26 stimuli with a duration of 300 ms) with an illumination intensity of <30mW/mm2, below the damage threshold of -100 mW/mm2 for blue light (Cardin et al., 2010). LPO electrical stimulation with single square pulses to elite inhibition in LH/PeF area was carried out 100ms before light stimulation (0.3 ms duration, and 20-50 µA intensity, delivered at 1 Hz; Cibertec Stimulator, Spain). In an off-line analysis, the single-unit activity during the light pulse in the first 50 ms was subtracted from the basal activity (50 ms before the light). Neurons that have a response of fewer than 0.2 spikes per stimulus were ruled out. Several stimuli were selected to avoid spontaneous oscillations' artifact. The stimuli have been considered a clean response, as long as there are more than six stimuli continuously. Basal unitary activity (light pulse stimulation) was compared with the second stimulation (LPO electrical stimulation + light pulse stimulation, with 100ms delay between them).

Statistics. Statistical analysis was performed using Graph Pad Prism 8 software (San Diego, CA, USA). The sample size for each experiment was chosen based on previous experience and considering a reduced use of animals. When data followed a normal distribution, we used student's t-test for comparing two groups (Welch's correction was used for data sets with unequal variance), and for more than two groups either 1-way, 2-way ANOVAs or 2-way RM ANOVAs followed by Tukey's or Sidak's multiple comparison test as a post hoc. For non-normally distributed data, we used the Mann Whitney U. All results are shown as mean ± standard error (SEM) and significant values as: *p<0.05; **p<0.01; ***p<0.001.

### Results and discussion

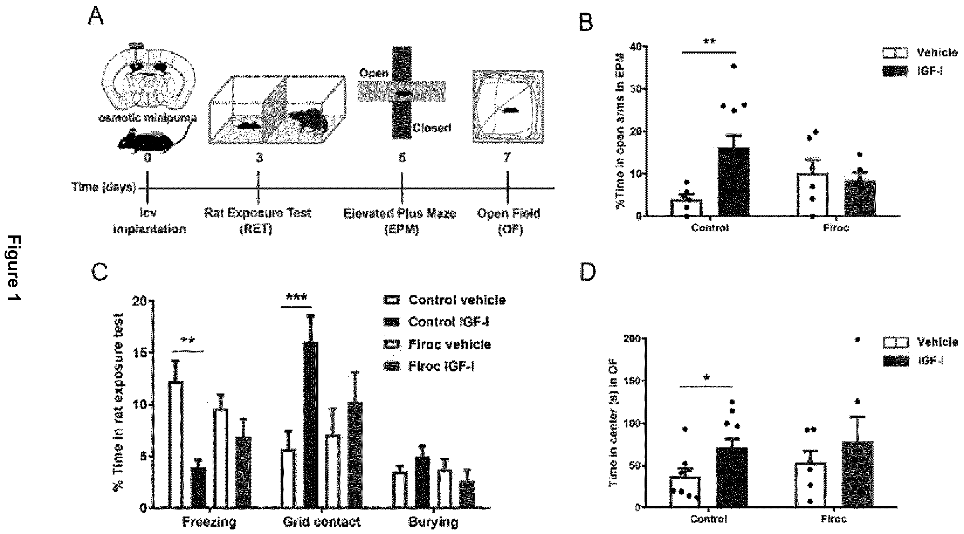

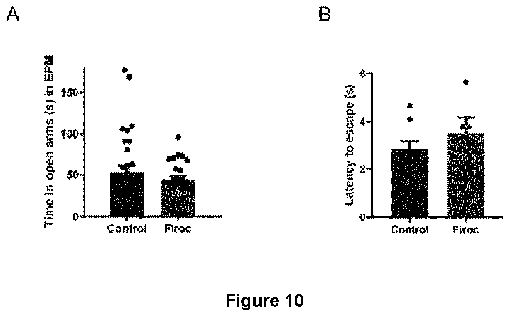

Anxiolytic actions of IGF-I involve orexin neurons. The present inventors recently found that IGF-I modulates the activity of orexin neurons (Zegarra-Valdivia et al., 2020). Since IGF-I reduces anxiety in mice exposed to a predator (Santi et al., 2018b), a natural anxiogenic stimulus (Blanchard et al., 2003), and inhibition of orexin attenuates anxiety responses to predator fear (Staples and Cornish, 2014), IGF-I (icv) was administered to mice expressing a truncated inactive IGF-IR in orexin neurons (Firoc mice) before exposure to a rat, and subsequently evaluated anxiety using the elevated plus maze (EPM), and the open field (Figure 1A). Reduced anxiety was observed in littermate controls treated with IGF-I, as compared to saline-treated ones, but not in Firoc mice, as reflected by time spent in the open arms of the EPM (Figure 1B). Other parameters measuring fear behavior during predator exposure (freezing and grid contacts), and time spent in the center of the open field arena after exposure to the rat, were also significantly ameliorated by IGF-I in control, but not in Firoc mice (Figure 1C, D). Importantly, naive Firoc mice show normal anxiety levels (Figure 10A), and normal latency to escape to an aversive electric shock (Figure 10B). Collectively, these observations suggest that orexin neurons are involved in modulation of coping behavior (i.e.: response to predator IGF-I modulates orexin responses to fear learning

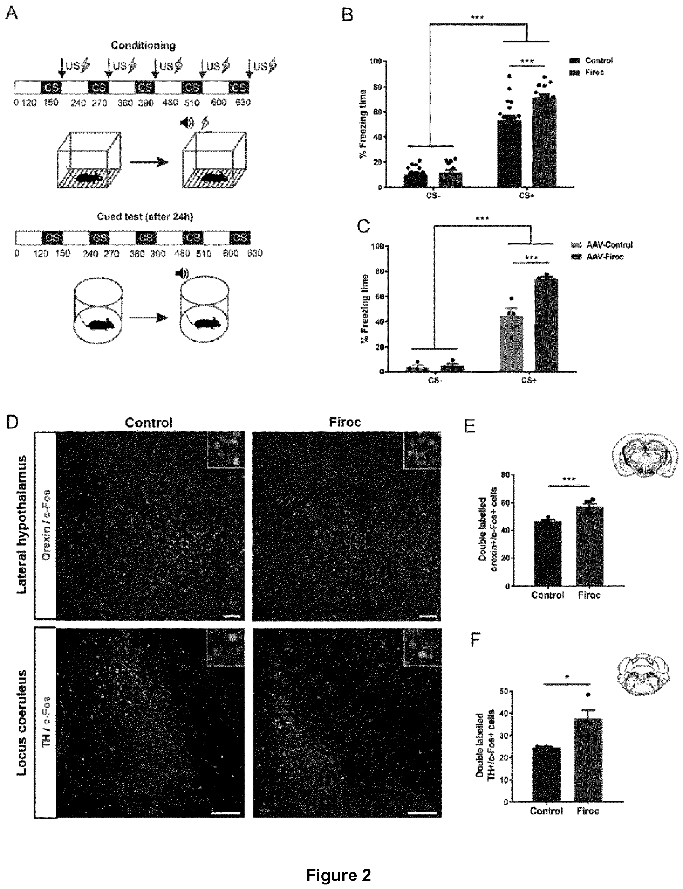

Based on the above observations, it was hypothesized that Firoc mice could show aberrant fear learning. Hence, they were submitted to fear conditioning (Figure 2A), and it was found that Firoc mice showed increased fear responses, as measured by time spent in freezing behavior (Figure 2B). To rule out developmental effects of IGF-IR inactivation in Firoc mice, AAV-Orexin-Cre virus was administered to adult floxed IGF-IR mice (AAV-Firoc mice) and their freezing behavior after the same protocol of fear learning followed in transgenic Firoc mice was determined. A similar increased freezing response was observed in AAV-Firoc mice, as compared to AAV-Control mice (Figure 2C).

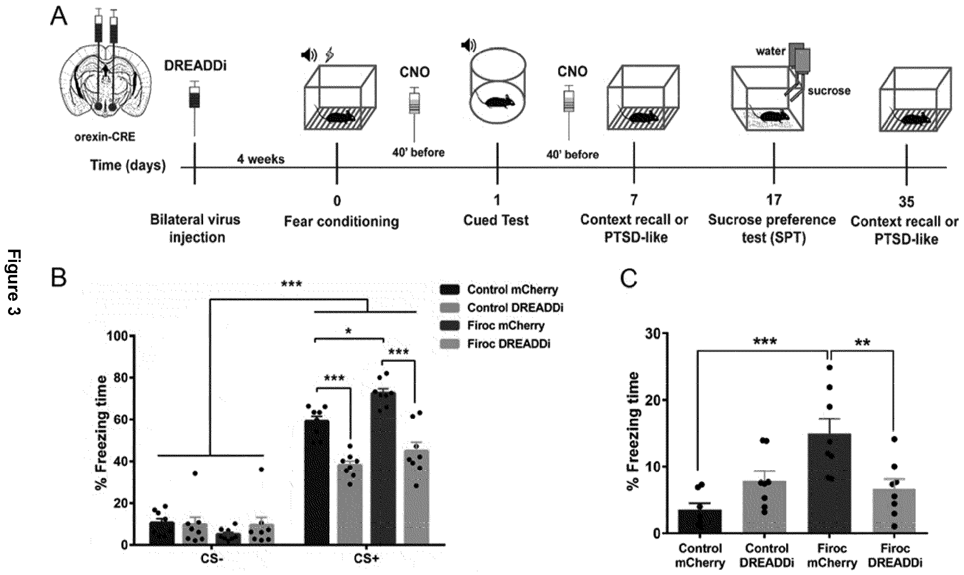

Firoc mice also showed increased number of activated orexin (double labeled c-fos+/orexin+ neurons; Figure 2D, upper panels, and Figure 2E), and locus coeruleus (LC) neurons (double labeled c-fos+/tyrosine hydroxylase+ cells, Figure 2D, lower panels and Figure 2F), a key downstream connection of orexin neurons in fear responses (Soya et al., 2017). Since these observations suggested increased activation of orexin neurons in Firoc mice submitted to fear conditioning, orexin activity after fear conditioning was inhibited using chemogenetic inhibition with Designer Receptors Exclusively Activated by Designer Drugs (DREADD). After bilateral injection of an inhibitory DREADD-Cre virus (hM4Di) into the lateral hypothalamus of Orexin-Cre (controls) and Firoc mice, CNO was administered to fear-conditioned mice 40 min before behavioral testing (Figure 3A), and it was found that Firoc mice normalized their fear responses (Figure 3B). Control littermates and Firoc mice injected with mCherry control virus show normal and exaggerated fear responses, respectively, as expected (Figure 3B). These data indicate that orexin neurons in Firoc mice submitted to fear learning are over-activated.

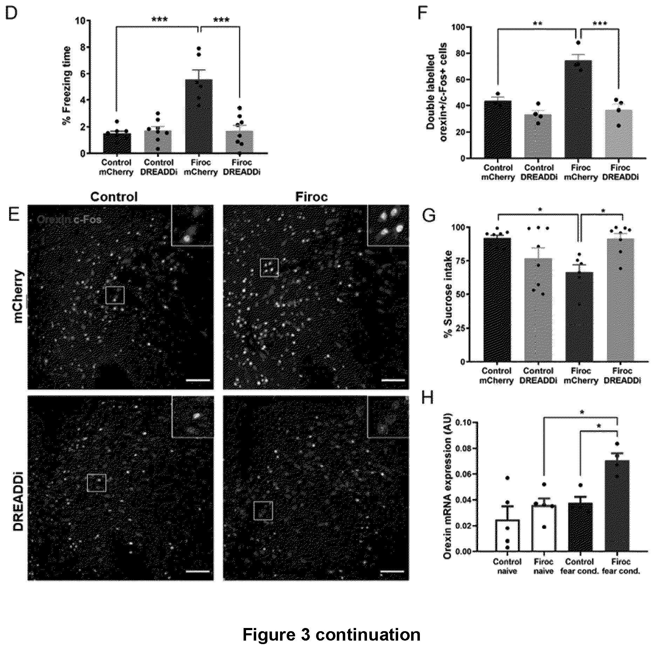

Over-activation of orexin neurons is involved in PTSD-like behavior. Abnormal fear learning is suggested to underlie PTSD (Shalev et al., 2017). Taking advantage that chemogenetic inactivation of orexin neurons in Firoc mice rescued exaggerated fear responses, the possible development of other PTSD-like traits and its attenuation with DREADD-mediated inhibition was studied using an hM4Di virus. Extinction of context-dependent fear responses (context recall) 1 week later, was markedly impaired in Firoc mice injected with mCherry virus, as compared to mCherry-injected littermate controls (Figure 3C). This indicates abnormal retention of learned fear, a trait seen in PTSD patients (Ross et al., 2017). However, after CNO administration, Firoc mice injected with hM4Di virus show normalized fear responses (Figure 3C). Since long-term fear memory is abnormally maintained in PTSD, fear responses in Firoc mice 5 were re-assessed weeks after fear learning and found them to be still abnormally elevated (Figure 3D). However, Firoc DREAAD mice treated with CNO 1 week after fear learning show normal freezing behavior in the fifth week (Figure 3D). Accordingly, c-fos expression in orexin neurons was still significantly greater 5 weeks after fear conditioning in control (mCherry) Firoc mice, but normal in Firoc DREAAD mice treated with CNO at week 1 (Figure 3E, F). Of note, while Firoc mice exhibited increased freezing at all times, extinction of the learned response also took place in them, albeit at a slower pace (compare Figures 3B-D).

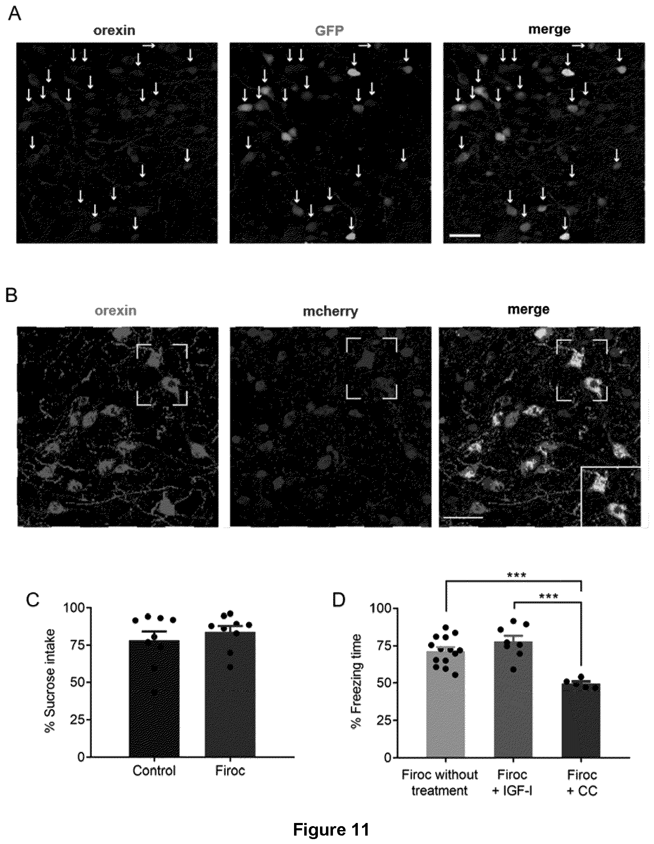

Firoc mice injected with mCherry virus also developed anhedonia (another trait of PTSD patients) after fear conditioning, as measured by the sucrose-preference test (Figure 3G). Anhedonia was also ameliorated in Firoc mice chemogenetically inhibited 1 week after fear-learning (Figure 3G). Naive Firoc mice did not show anhedonia (Figure 11C). Interestingly, Firoc mice also showed higher levels of orexin in the hypothalamus after fear conditioning (Figure 3H).

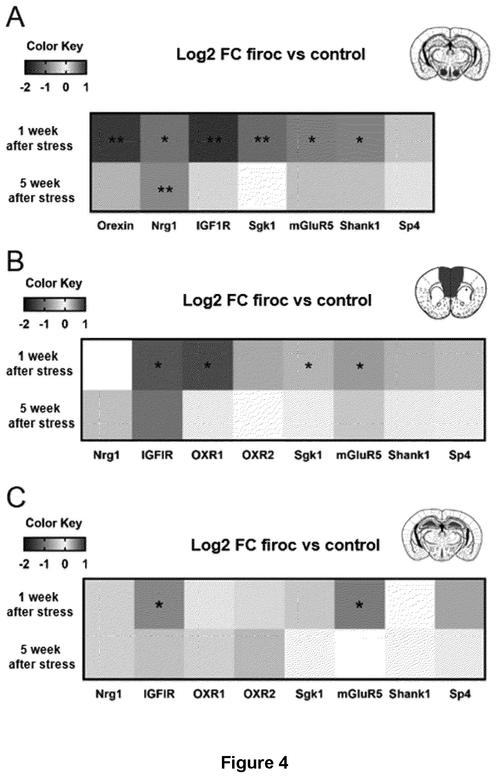

A PTSD-like molecular signature in Firoc mice after fear learning. A number of molecular changes have been described in PTSD patients and rodent models (DePierro et al., 2019). Acute changes in the latter are proposed to be adaptive (Richter-Levin et al., 2019), whereas those seen in patients are more likely reflecting pathological alterations, as they are assessed post-mortem, well after the disease is established. To further validate Firoc mice as a potential model of PTSD, changes in a selected panel of genes (Table 2) in medial prefrontal cortex (mPFC), hippocampus and hypothalamus were determined at 1 and 5 weeks after fear-learning. Candidate genes were selected in relation to previously published association with PTSD and/or orexin function. Most prominent changes were seen 1 week after fear learning in the hypothalamus (Figure 4A), followed by the mPFC (Figure 4B) and hippocampus (Figure 4C). Only mGluR5 and IGF-IR changed in the three regions studied. Most changes were short-lived, as 5 weeks later no changes were seen except for hypothalamic Nrg1, involved in synaptic plasticity and PTSD (Table 2). mRNA levels of dynorphin, co-expressed by orexin neurons (Chou et al. 2001), were not changed (not shown). Indeed, dynorphin transmission shows functional characteristics independent of orexin (Muschamp et al. 2014). No significant changes were found in POMC mRNA levels in hypothalamus (data not shown).

**Table 2. Gene profiling in Firoc mice.**

| **Gene** | **Function** | **References** |
|---|---|---|
| Orexin | Involved in PTSD | (Cohen et al. 2020) |
| Orexin 1/2 Receptor | Involved in PTSD | (Han et al. 2020) |
| | | (Flores et al. 2014) |
| | | (Salehabadi et al. 2020) |
| Sgk1 | Involved in PTSD | (Licznerski et al., 2015) |
| | | (Torrisi et al. 2020) |
| | | (Duman et al. 2018) |
| | Memory formation | (von Hertzen et al. 2005) |
| | Role in HPA activity | (Dattilo et al. 2020) |
| Nrg1 | Glutamatergic/GABAergic synapses | (Li et al., 2007) |
| | | (Chen et al. 2010) |
| | | (Ting et al. 2011) |
| | | (Wang et al. 2021) |
| | | (Lu et al. 2014) |
| | Involved in PTSD and fear memory | (Uddin et al. 2018) (Chen et al. 2017) |
| | Role in HPA activity | (Taylor et al. 2011) |
| Sp4 | Glutamatergic/GABAergic synapses | (Priya et al., 2014) |
| | | (Nair et al., 2016) |
| mGluR5 | Involved in PTSD and fear extinction | (Holmes et al. 2020) |
| | | (Sethna et al. 2016) |
| | | (Kordestani-Moghadam et al. 2020) |
| Shank1 | Involved in PTSD | (Holmes et al. 2020) |
| Dynorphin | Hypothalamic neuropeptide co-expressed in orexin neurons | (Chou et al. 2001) |
| POMC | Hypothalamic neuropeptide | (Toda et al. 2017) |

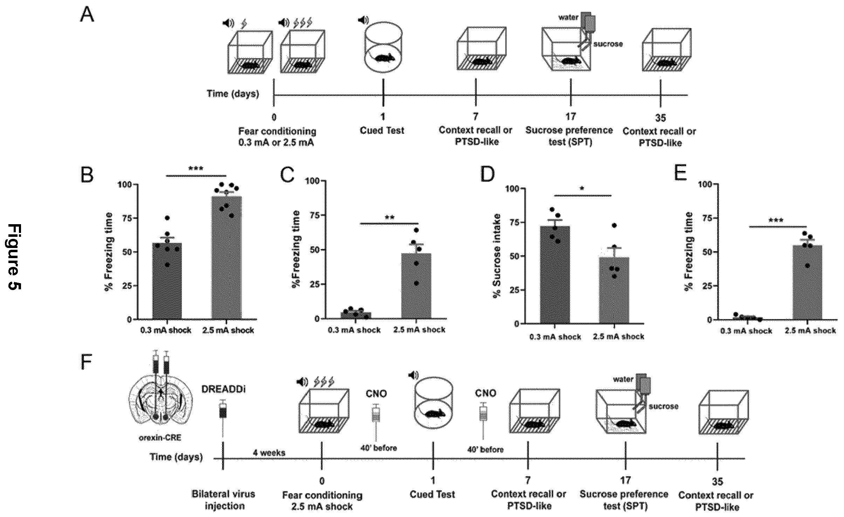

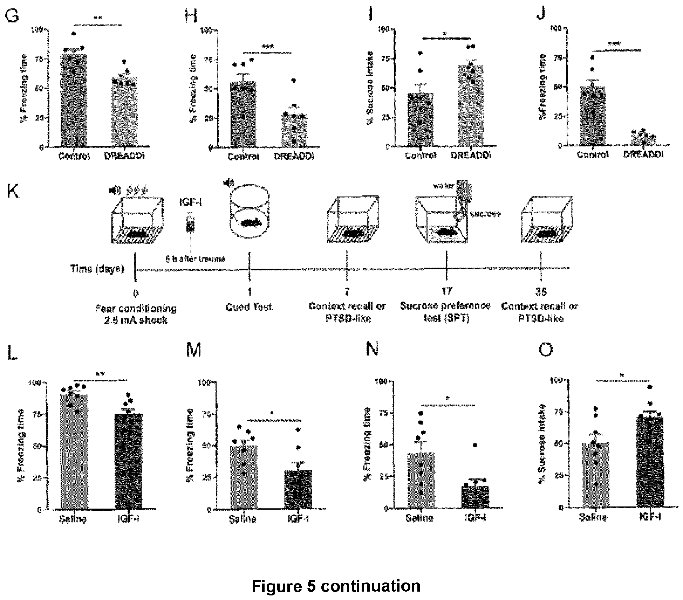

An IGF-l/orexin link in PTSD-like behavior. Since these observations confirm previous ones indicating that orexin neurons are involved in PTSD (Flores et al. 2014; Soya et al. 2017; Cohen et al. 2020), PTSD-like behavior was induced in control mice to determine whether orexin neurons are also involved. After fear learning through exposure to a 2.5 mA electric shock, wild type mice display exaggerated fear behavior as compared to mice submitted to 0.3 mA electric shocks (Figure 5A,B). hM4Di or mCherry viruses were injected to orexin-Cre mice submitted to the 2.5 mA shock protocol. Increased freezing was ameliorated by DREAAD inhibition of orexin neurons after administration of CNO (Figure 5G). Protracted, PTSD-like behavioral responses at 1 and 5 weeks after fear learning were also attenuated by DREADD inhibition in these mice (Figure 5G,H,J), including anhedonia (Figure 5I), as previously seen in Firoc mice.

Next, the same experimental protocol was used to determine whether IGF-I attenuates PTSD-like responses in wild type control mice. Indeed, wild type mice that develop exaggerated freezing after fear learning with 2.5 mA shocks, show normal freezing responses after treatment with systemic IGF-I (ip, 1 µg/kg, 6 hours after training; Figure 5L). Protective effects of systemic IGF-I administration were seen for at least 1 and 5 weeks (Figure 5M, O). Anhedonia induced by the PTSD-eliciting protocol in wild type mice was also ameliorated by IGF-I treatment (Figure 5N). However, Firoc mice displaying exaggerated fear responses after fear learning with 0.3 mA electric shocks were unresponsive to IGF-I treatment (Figure 11D).

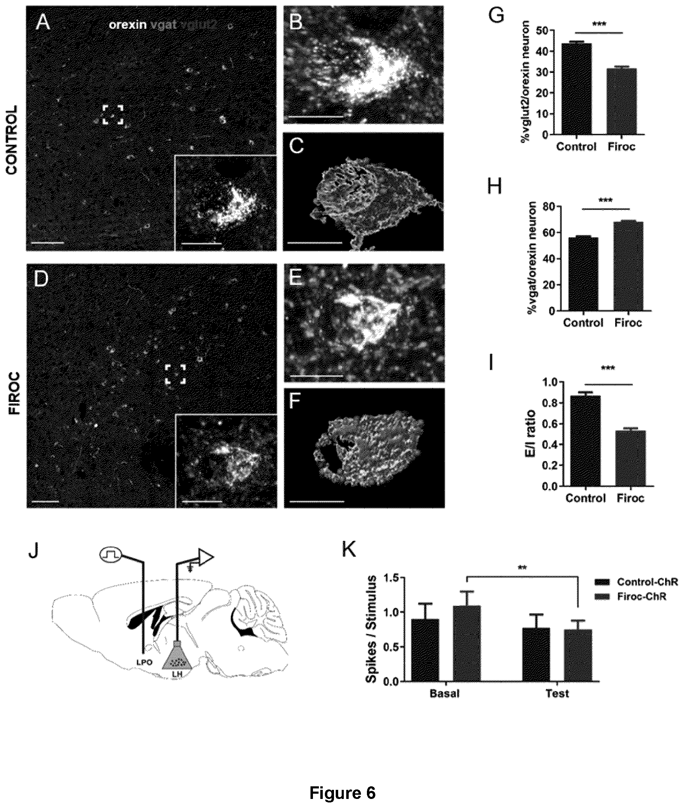

Excitatory/inhibitory balance in orexin neurons is modulated by IGF-I. Orexin neurons show a highly plastic excitatory/inhibitory input that can be hormonally regulated (Horvath and Gao, 2005). Thus, it was determined whether the absence of IGF-IR activity in orexin neurons of Firoc mice affects Glutamatergic and GABAergic (Glu/GABA) inputs onto them. Orexin immunocytochemistry combined with Vgat or Vglut2 were used to identify GABAergic or glutamatergic synaptic puncta, respectively. Firoc mice showed decreased number of Vglut2 puncta/orexin cell and increased Vgat puncta/orexin cell (Figure 6A-H), resulting in a decreased Glu/GABA ratio onto orexin neurons (Figure 6I).

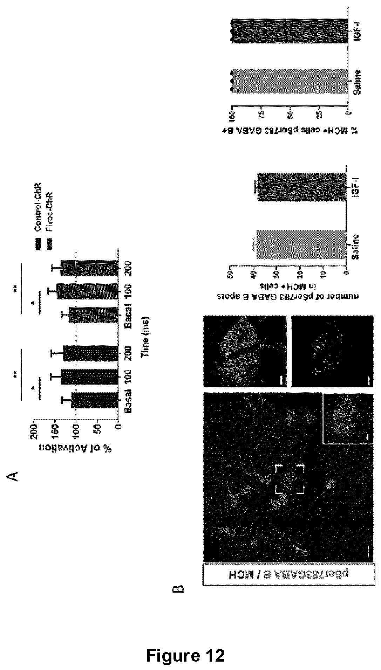

To assess whether the activity of orexin neurons was inhibited in Firoc animals as compared to control littermates, the effect of electrical stimulation of the lateral pre-optic area (LPO) on the activity of orexin neurons of the lateral hypothalamus (LH; Figure 6J) was studied, as these neurons inhibit them (Saito et al., 2013). Firoc (Firoc-ChR) and littermates (Or-ChR, control) mice expressing channelrhodopsin in orexin neurons were identified through optogenetic stimulation (Figure 6J), as described (Zegarra-Valdivia et al., 2020), and used for the study. Short-lasting blue LED stimuli induced an increase in spike firing in orexinergic neurons of Or-ChR mice at 100ms (134.9 ± 24.58 spikes, 26 stimuli; Figure 12A) over basal conditions (111.1 ± 22.16 spikes, 26 stimuli; n=19-19/group; **p<0.0032), as well as at 200ms (130.7 ± 27.87 spikes, 26 stimuli; 19-19/group; *p<0.044). In Firoc-ChR mice, the short-lasting blue LED stimuli induced an increase at 100ms (145.4 ± 20.92 spikes, 26 stimuli) over basal conditions (117.1 ± 16.47 spikes, 26 stimuli; n=27-27/group; **p<0.0034), as well as at 200ms (136.4 ± 20.89 spikes, 26 stimuli; 19-19/group; *p<0.016). Thus, no differences were seen between groups. The effect lasted 200ms, recovering baseline activity later, even though the blue-light pulse lasted 300ms. After optogenetically identifying orexin neurons, LPO was stimulated before the optogenetic stimulation. Under basal conditions, light pulses elicited 0.9±0.22 spikes/50 ms in orexinergic neurons (n= 11) of control animals, while the same light pulse elicited 1.1±0.19 spikes/50 ms (n= 14) in orexinergic neurons of Firoc-ChR animals. After that, electrical stimulation of the LPO 100ms before light onset induced a reduction of responses to light stimulation significantly larger in Firoc-ChR orexinergic neurons than in littermates (Figure 6K). In control animals, the light response was slightly reduced to 0.78±0.19 spikes/50 ms (13%; p=0.1135); however, the reduction was more extensive in Firoc-ChR animals, reaching statistical significance (31%: 0.76±0.12 spikes/50 ms; p=0.0030). Collectively, these changes agree with previous observations of decreased orexin responses to afferent stimulation in Firoc mice (Zegarra-Valdivia et al., 2020), and confirm that lack of IGF-IR in orexin neurons enhances inhibitory transmission onto them.

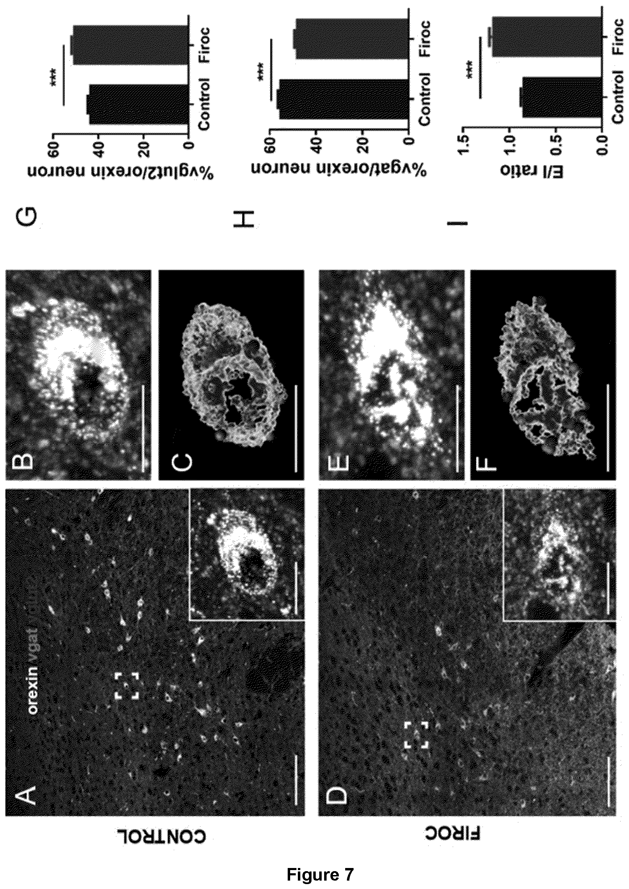

As organization of synaptic architecture of orexin neurons is highly experience-dependent (Gao and Wang, 2010), it was assessed whether fear learning in Firoc mice induces synaptic remodeling that could explain the observed upregulation of orexin activity. Significantly, upon fear-learning, Firoc mice showed increased hypothalamic Nrg1 expression (Figure 4A), that promotes glutamatergic transmission (Li et al., 2007). This agrees with the observed increase of Vglut2 puncta in orexin neurons of Firoc mice undergoing fear learning (Figure 7A-C, G). At the same time, decreased Vgat puncta in orexin neurons was observed (Figure 7D-F, H). Overall, the Glu/GABA ratio was increased in Firoc mice submitted to fear learning (Figure 7I). Increased E/I balance fully agrees with the observed enhanced freezing responses of Firoc mice.

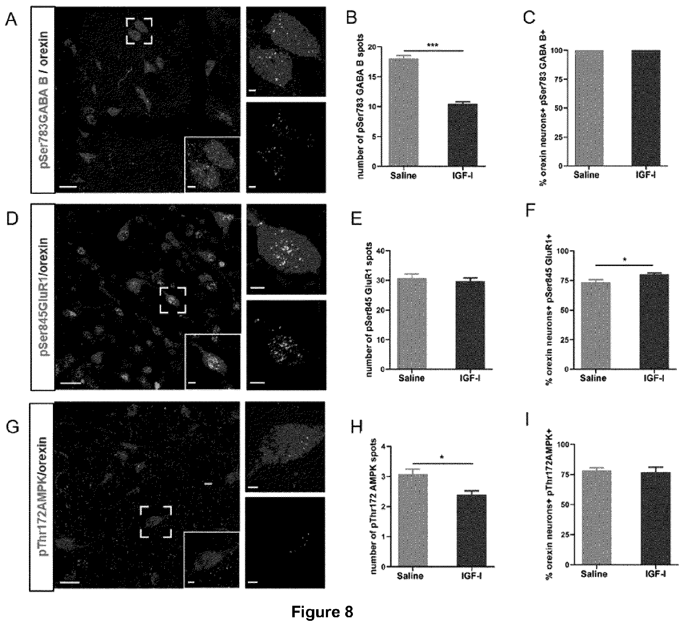

Collectively, the above results indicate that the absence of a functional IGF-IR in orexin neurons imbalances glutamatergic and GABAergic inputs. To confirm an effect of IGF-I on neurotransmission in orexin neurons, IGF-I (ip, 1 µg/gr, 2 hours before sacrifice) was administered to wild type mice and determined immunostaining in orexin cells of either pSer783/893GABA(B)R or pSer845GluR1, markers of active GABAergic (Kuramoto et al., 2007) and Glutamatergic (Cheng et al., 2020) receptors, respectively. While all orexin neurons presented pSer783/893 GABA(B) R immunoreactivity, IGF-I significantly decreased the number of pSer783/893 GABA(B) R puncta in them (Figure 8A-C), indicating decreased postsynaptic GABA(B) R activity. At the same time, IGF-I modestly, but significantly increased the number of double labeled orexin/pSer845GluR1 neurons (Figure 11B), indicating increased number of orexin neurons with active postsynaptic glutamate receptors, although the amount of pSer845GluR1 staining per cell was not affected (Figure 8D-F). Since AMPK stimulates GABAR function (Kuramoto et al., 2007), and Akt, that is downstream of IGF-I signaling, inhibits AMPK (Kovacic et al., 2003), the levels of pThr172 AMPKα immunoreactivity (an indicator of activation of AMPK (Deng et al., 2016) were determined in orexin neurons after IGF-I administration and found them decreased (Figure 8G-I). Since IGF-I did not affect pSer783/893 GABA(B) R immunoreactivity in MCH neurons (Figure 12B), a neighboring population known to interact with orexin neurons (Hung et al., 2020), the latter appear to be the target of IGF-I in modulating fear behavior.

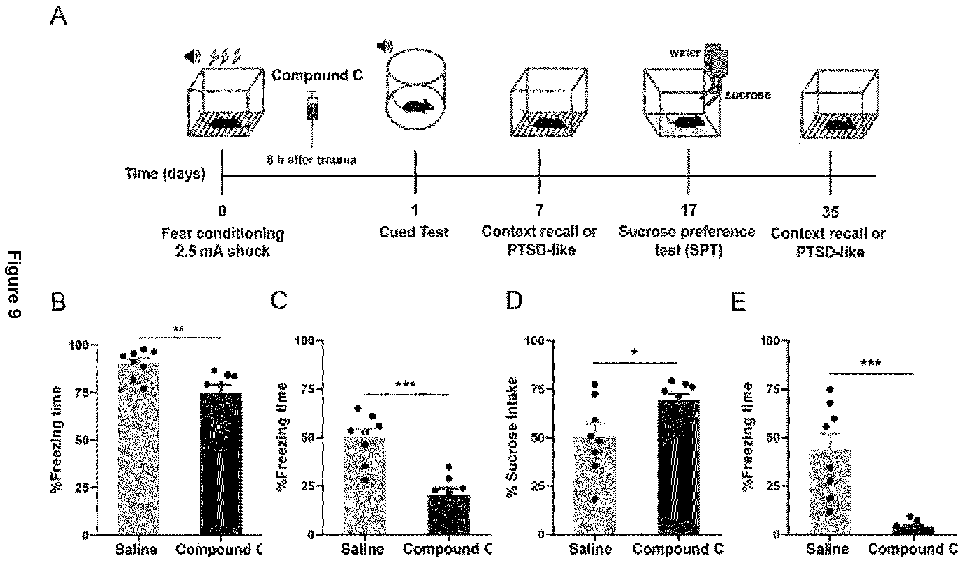

To confirm a role of AMPK in the actions of IGF-I onto orexin neurons, the activity of the molecule was inhibited by ip administration of Compound C (CC). Indeed, CC-treated wild type mice submitted to the PTSD protocol showed normalized fear behavior, thus mimicking the effects of IGF-I (Figure 9A-E). Of note, Firoc mice treated with CC also show normal freezing behavior, reinforcing AMPK inhibition is downstream of IGF-I signaling (Suppl Fig 11D)

### Discussion

Attenuation by IGF-I of stress responses to trauma (predator exposure) was abrogated in Firoc mice lacking a functional IGF-IR in orexin neurons, indicating that IGF-I signaling onto orexin neurons is important for appropriate coping with stressful stimuli. In addition, Firoc mice developed PTSD-like behaviors (Pietrzak et al., 2014) after fear conditioning, displayed molecular changes in various brain areas reminiscent of changes seen in other experimental models of PTSD, and showed a decreased VGlut2/Vgat ratio onto the soma of orexin neurons, suggesting reduced pre-synaptic glutamatergic (excitatory) inputs and increased pre-synaptic GABAergic (inhibitory) inputs. Accordingly, orexin neurons of Firoc mice showed greater inhibition in response to LPO afferent stimulation. Similar excitatory/inhibitory (E/I) input re-arrangements onto orexin neurons has been seen during the sleep phase in mice (Laperchia et al., 2017), suggesting a functional impact of the observed E/I plasticity, which agrees with increased δ band activity in the electroencephalogram of naive Firoc mice (Zegarra-Valdivia et al., 2020). Conversely, IGF-I slightly favors glutamatergic transmission onto orexin neurons, as indicated by moderately increased active post-synaptic glutamatergic receptors (increased double labeled orexin/pSer845GluR1 neurons), whereas GABAergic transmission is downregulated, as indicated by reduced active post-synaptic GABA receptors (decreased pSer783/893 GABA(B) receptor and pThr172 AMPKα staining in orexin neurons). The intermediary role of AMPK in modulatory actions of IGF-I was confirmed using CC, an AMPK inhibitor that was able to mimic the effect of IGF-I in vivo, and by higher pThr172 AMPKα staining in orexin neurons of Firoc mice.

A reduced E/I ratio in the hippocampal neurons of serum IGF-I deficient mice was previously observed by us (Trejo et al., 2007), suggesting a broader modulation by IGF-I of E/I balance. Indeed, this same modulatory effect on E/I transmission has recently been postulated for invertebrate insulin peptides (McCulloch et al., 2020), and confirmed by the inventors in the mouse cerebral cortex (Noriega et al, submitted). A recent report in obese mice show also a similar E/I imbalance in orexin neurons leading to a mood phenotype that is reminiscent of the Firoc mice (Tan et al., 2020), reinforcing the notion that imbalanced inhibition of orexin neurons hinders appropriate responses to stress (this manuscript), and reward (Zegarra-Valdivia et al, submitted). Altogether, these results expand previous observations suggesting that IGF-I is a neuroendocrine regulator of neuronal plasticity in different brain areas (reviewed in (Fernandez and Torres-Aleman, 2012), affecting in this case coping behavior.

IGF-IR in orexin neurons modulates both acquired (fear learning), and innate fear (predator exposure) responses, providing a specific mechanism for resilience/ vulnerability to emotional trauma. Thus, the PTSD-like behavior of both Firoc mice or wild type mice submitted to 2.5 mA electric shocks, is normalized by early chemogenetic inhibition of orexin neurons, which supports the use of orexin receptor antagonists for treatment of this condition (Flores et al., 2015; Soya and Sakurai, 2018). Conversely, recent observations pose orexin activation in experimental PTSD as a mechanism of resilience (Cohen et al., 2020), and support the use of orexin agonists for treatment of PTSD (Han et al., 2020). These two apparently opposing observations may be reconciled by the present findings. Since tempered responses to stress require appropriate IGF-I signaling onto orexin neurons, IGF-I may modulate orexin activity in response to fear stimuli to enable coping responses, as suggested by Cohen et al (Cohen et al., 2020). Indeed, after a strong fear stimulus, PTSD-like behavior in wild type mice was corrected by early administration of IGF-I, a procedure that was not effective in Firoc mice. In turn, when IGF-I regulation is compromised (as in Firoc mice), orexin activity becomes maladaptive; i.e.: greater expression of orexin, increased number of double-labelled c-fos+/orexin+ and c-fos+/TH+ cells, and increased E/I ratio onto orexin neurons are found in Firoc mice after fear conditioning, as compared to littermates. Thus, inappropriate activation of the orexin-LC circuitry leading to aberrant fear behavior (Soya et al., 2017) would be produced by insufficient IGF-I balancing. The reason why PTSD-like behavior is elicited in wild type mice in response to a stronger stress (2.5 mA electric shocks), and ameliorated by systemic IGF-I administration may be related to the fact that stress inhibits the passage of IGF-I into the brain (Fernandez et al., 2018). Again, inappropriate IGF-I regulation of orexin activity would underlie this process.

This novel interaction between IGF-I and orexin activity has allowed the inventors to document that early intervention abrogates long-term PTSD traits such as impaired fear extinction and anhedonia after 5 weeks of fear-conditioning. This furthers previous observations that early treatment provides better therapeutic efficacy in PTSD (Stein and Rothbaum, 2018). Indeed, early inhibition of excess orexinergic activity in Firoc mice by DREADDi or in wild type mice submitted to intense fear learning with either DREADDi, systemic IGF-I administration, or CC, corrected PTSD-like traits. The latter opens the possibility of using AMPK inhibitors for treatment of PTSD.

The fact that a pleiotropic neurotrophic factor such as IGF-I (Fernandez and Torres-Aleman, 2012) modulates the activity of orexin neurons, considered also a multitasking system (Sakurai, 2014), contributers to explain the diversity of actions of IGF-I in the brain, including regulation of mood. The results presented herein widen the number of potential mechanisms whereby IGF-I intervenes in mood homeostasis, including coping responses through the orexinergic system, which emphasizes the system level (i.e.: mood) of actions of this growth factor in the brain.

### REFERENCES

Blanchard DC, Griebel G, Blanchard RJ (2003) The Mouse Defense Test Battery: pharmacological and behavioral assays for anxiety and panic. Eur J Pharmacol 463:97-116.
Cardin JA, Carlen M, Meletis K, Knoblich U, Zhang F, Deisseroth K, Tsai LH, Moore Cl (2010) Targeted optogenetic stimulation and recording of neurons in vivo using cell-type-specific expression of Channelrhodopsin-2. Nat Protoc 5:247-254.
Cheng W, Siedlecki-Wullich D, Català-Solsona J, Fábregas C, Fadó R, Casals N, Sole M, Unzeta M, Saura CA, Rodriguez-Alvarez J, Miñano-Molina AJ (2020) Proteasomal-Mediated Degradation of AKAP150 Accompanies AMPAR Endocytosis during cLTD. eNeuro 7.
Cohen S, Matar MA, Vainer E, Zohar J, Kaplan Z, Cohen H (2020) Significance of the orexinergic system in modulating stress-related responses in an animal model of post-traumatic stress disorder. Translational Psychiatry 10:10.
Chen YJ, Zhang M, Yin DM, et al. ErbB4 in parvalbumin-positive interneurons is critical for neuregulin 1 regulation of long-term potentiation. Proc Natl Acad Sci USA 107, 21818-21823 (2010)
Chen YH, Lan YJ, Zhang SR, et al. ErbB4 signaling in the prelimbic cortex regulates fear expression. Transl Psychiatry. 7, e1168 (2017)
Chou TC, Lee C E, Lu J, Elmquist J K, Hara J, Willie J T, Beuckmann C T, Chemelli R M, Sakurai T, Yanagisawa M, Saper C B, Scammell T E (2001). Orexin (hypocretin) neurons contain dynorphin. J Neurosci 21(19):RC168.
Dattilo V, Amato R, Perrotti N, Gennarelli M (2020) The Emerging Role of SGK1 (Serum- and Glucocorticoid-Regulated Kinase 1) in Major Depressive Disorder: Hypothesis and Mechanisms. Front Genet. 11:826.
de Leeuw CN, Dyka FM, Boye SL, Laprise S, Zhou M, Chou AY, Borretta L, Mclnerny SC, Banks KG, Portales-Casamar E, Swanson MI, D'Souza CA, Boye SE, Jones SJ, Holt RA, Goldowitz D, Hauswirth WW, Wasserman WW, Simpson EM (2014) Targeted CNS Delivery Using Human MiniPromoters and Demonstrated Compatibility with Adeno-Associated Viral Vectors. Mol Ther Methods Clin Dev 1:5.
Deng M, Yang X, Qin B, Liu T, Zhang H, Guo W, Lee SB, Kim JJ, Yuan J, Pei H, Wang L, Lou Z (2016) Deubiquitination and Activation of AMPK by USP10. Mol Cell 61:614-624.
Duman RS, Girgenti MJ (2019) Molecular and cellular studies of PTSD: Postmortem transcriptome analysis and novel therapeutic targets. J Neurosci Res. Mar;97(3):292-299.
Fernandez AM, Torres-Aleman I (2012) The many faces of insulin-like peptide signalling in the brain. Nat Rev Neurosci 13:225-239.
Fernandez AM, Santi A, Torres Aleman I (2018) Insulin Peptides as Mediators of the Impact of Life Style in Alzheimer's disease. Brain plasticity (Amsterdam, Netherlands) 4:3-15.
Flores Á, Saravia R, Maldonado R, Berrendero F (2015) Orexins and fear: implications for the treatment of anxiety disorders. Trends in Neurosciences 38:550-559.
Flores Á, Valls-Comamala V, Costa G, Saravia R, Maldonado R, Berrendero F (2014) The hypocretin/orexin system mediates the extinction of fear memories. Neuropsychopharmacology official publication of the American College of Neuropsychopharmacology 39:2732-2741.
Fogarty MJ, Hammond LA, Kanjhan R, Bellingham MC, Noakes PG (2013) A method for the three-dimensional reconstruction of Neurobiotin-filled neurons and the location of their synaptic inputs. Front Neural Circuits 7:153.
Gao XB, Wang AH (2010) Experience-dependent plasticity in hypocretin/orexin neurones: re-setting arousal threshold. Acta Physiol (Oxf) 198:251-262.
Han D, Han F, Shi Y, Zheng S, Wen L (2020) Mechanisms of Memory Impairment Induced by Orexin-A via Orexin 1 and Orexin 2 Receptors in Post-traumatic Stress Disorder Rats. Neuroscience 15, 126-136.
Holmes SE, Girgenti MJ, Davis MT, Pietrzak RH, DellaGioia N, Nabulsi N, Matuskey D, Southwick S, Duman RS, Carson RE, Krystal JH, Esterlis I (2017)Traumatic Stress Brain Study Group. Altered metabotropic glutamate receptor 5 markers in PTSD: In vivo and postmortem evidence. Proc Natl Acad Sci U S A. 114, 8390-8395.
Horvath TL, Gao XB (2005) Input organization and plasticity of hypocretin neurons: possible clues to obesity's association with insomnia. Cell Metab 1:279-286.
Hung CJ, Ono D, Kilduff TS, Yamanaka A (2020) Dual orexin and MCH neuron-ablated mice display severe sleep attacks and cataplexy. Elife 9.
Kovacic S, Soltys CL, Barr AJ, Shiojima I, Walsh K, Dyck JR (2003) Akt activity negatively regulates phosphorylation of AMP-activated protein kinase in the heart. J Biol Chem 278:39422-39427.
Kordestani-Moghadam P, Nasehi M, Khodagholi F, Vaseghi S, Zarrindast MR, Khani M (2020) The fluctuations of metabotropic glutamate receptor subtype 5 (mGluR5) in the amygdala in fear conditioning model of male Wistar rats following sleep deprivation, reverse circadian and napping. Brain Res. 11734:146739.
Kuramoto N, Wilkins ME, Fairfax BP, Revilla-Sanchez R, Terunuma M, Tamaki K, lemata M, Warren N, Couve A, Calver A, Horvath Z, Freeman K, Carling D, Huang L, Gonzales C, Cooper E, Smart TG, Pangalos MN, Moss SJ (2007) Phospho-dependent functional modulation of GABA(B) receptors by the metabolic sensor AMP-dependent protein kinase. Neuron 53:233-247.
Lecca S, Meye FJ, Trusel M, Tchenio A, Harris J, Schwarz MK, Burdakov D, Georges F, Mameli M (2017) Aversive stimuli drive hypothalamus-to-habenula excitation to promote escape behavior. Elife 6.
Lu Y, Sun XD, Hou FQ, et al. (2014) Maintenance of GABAergic activity by neuregulin 1-ErbB4 in amygdala for fear memory. Neuron. 84, 835-846.
Matsuki T, Nomiyama M, Takahira H, Hirashima N, Kunita S, Takahashi S, Yagami K, Kilduff TS, Bettler B, Yanagisawa M, Sakurai T (2009) Selective loss of GABA(B) receptors in orexin-producing neurons results in disrupted sleep/wakefulness architecture. Proc Natl Acad Sci U S A 106:4459-4464.
Mayeaux DJ, Tandle SM, Cilano SM, Fitzharris MJ (2015) Progesterone After Estradiol Modulates Shuttle-Cage Escape by Facilitating Volition. J Exp Neurosci 9:19-26.
McCulloch KA, Zhou K, Jin Y (2020) Neuronal transcriptome analyses reveal novel neuropeptide modulators of excitation and inhibition imbalance in C. elegans. PLoS One 15:e0233991.
Muschamp JW, Hollander JA, Thompson JL, Voren G, Hassinger LC, Onvani S, Kamenecka TM, Borgland SL, Kenny PJ, Carlezon WA, Jr (2014). Hypocretin (orexin) facilitates reward by attenuating the antireward effects of its cotransmitter dynorphin in ventral tegmental area. Proceedings of the National Academy of Sciences of the United States of America, 111(16), E1648-E1655.
Pietrzak RH, el-Gabalawy R, Tsai J, Sareen J, Neumeister A, Southwick SM (2014) Typologies of posttraumatic stress disorder in the U.S. adult population. Journal of Affective Disorders 162:102-106.
Ross DA, Arbuckle MR, Travis MJ, Dwyer JB, van Schalkwyk GI, Ressler KJ (2017) An Integrated Neuroscience Perspective on Formulation and Treatment Planning for Posttraumatic Stress Disorder: An Educational Review. JAMA Psychiatry 74:407-415.
Salehabadi S, Abrari K, Elahdadi Salmani M, Nasiri M, Lashkarbolouki T (2020) Investigating the role of the amygdala orexin receptor 1 in memory acquisition and extinction in a rat model of PTSD. Behav Brain Res. 384, 112455
Sakurai T (2014) The role of orexin in motivated behaviours. Nat Rev Neurosci 15:719-731.
Santi A, Genis L, Torres Aleman I (2018a) A Coordinated Action of Blood-Borne and Brain Insulin-Like Growth Factor I in the Response to Traumatic Brain Injury. Cereb Cortex 28:2007-2014.
Santi A, Bot M, Aleman A, Penninx BWJH, Aleman IT (2018b) Circulating insulin-like growth factor I modulates mood and is a biomarker of vulnerability to stress: from mouse to man. Translational Psychiatry 8:142.
Sethna F, Wang H (2016) Acute inhibition of mGluR5 disrupts behavioral flexibility. Neurobiol Learn Mem. 130, 1-6.
Shalev A, Liberzon I, Marmar C (2017) Post-Traumatic Stress Disorder. New England Journal of Medicine 376:2459-2469.
Soya S, Sakurai T (2018) Orexin as a modulator of fear-related behavior: Hypothalamic control of noradrenaline circuit. Brain research: 146037-146037.
Soya S, Takahashi TM, McHugh TJ, Maejima T, Herlitze S, Abe M, Sakimura K, Sakurai T (2017) Orexin modulates behavioral fear expression through the locus coeruleus. Nat Commun 8:1606.
Staples LG, Cornish JL (2014) The orexin-1 receptor antagonist SB-334867 attenuates anxiety in rats exposed to cat odor but not the elevated plus maze: an investigation of Trial 1 and Trial 2 effects. Horm Behav 65:294-300.
Tan Y, Hang F, Liu ZW, Stoiljkovic M, Wu M, Tu Y, Han W, Lee AM, Kelley C, Hajos M, Lu L, de Lecea L, de Araujo I, Picciotto M, Horvath TL, Gao XB (2020) Impaired hypocretin/orexin system alters responses to salient stimuli in obese male mice. J Clin Invest.
Taylor SB, Taylor AR, Markham JA, Geurts AM, Kanaskie BZ, Koenig JI (2011) Disruption of the neuregulin 1 gene in the rat alters HPA axis activity and behavioral responses to environmental stimuli. Physiol Behav. 104, 205-214.
Trejo JL, Piriz J, Llorens-Martin MV, Fernandez AM, Bolos M, LeRoith D, Nunez A, Torres-Aleman I (2007) Central actions of liver-derived insulin-like growth factor I underlying its pro-cognitive effects. Mol Psychiatry 12:1118-1128.
John W. Muschamp, Jonathan A. Hollander, Jennifer L. Thompson, George Voren, Linda C. Hassinger, Sara Onvani, Theodore M. Kamenecka, Stephanie L. Borgland, Paul J. Kenny, and William A. Carlezon Jr. (2014). Hypocretin (orexin) facilitates reward by attenuating the antireward effects of its cotransmitter dynorphin in ventral tegmental area. PNAS 111 (16) E1648-E1655.
Zegarra-Valdivia JA, Pignatelli J, Fernandez de Sevilla ME, Fernandez AM, Munive V, Martinez-Rachadell L, Nuñez A, Torres Aleman I (2020) Insulin-like growth factor I modulates sleep through hypothalamic orexin neurons. Faseb j.

## Claims

1. An AMP-activated protein kinase (AMPK) inhibitor or a pharmaceutical composition comprising said inhibitor in a therapeutically effective amount for use in the treatment and/or prevention of post-traumatic stress disorder (PTSD) in a subject.

2. The AMPK inhibitor or composition for use according to claim 1, wherein the AMPK inhibitor is selected from the group consisting of:
(i) Insulin-like Growth Factor I (IGF-I);
(ii) a small molecule; and
(iii) an inhibitor of *AMPK* gene expression.

3. The AMPK inhibitor or composition for use according to any one of the preceding claims, wherein the inhibitor is a small molecule.

4. The AMPK inhibitor or composition for use according to any one of the preceding claims, wherein the inhibitor is 6-[4-(2-Piperidin-1-yl-ethoxy)-phenyl)]-3-pyridin-4-yl-pyrrazolo[1,5-a]-pyrimidine.

5. The AMPK inhibitor or composition for use according to any one of the preceding claims, wherein the pharmaceutical composition further comprises a compound for the treatment of PTSD in a subject.

6. The AMPK inhibitor or composition for use according to any one of the preceding claims, wherein the subject is a human subject.

7. The AMPK inhibitor or composition for use according to any one of the preceding claims, wherein the AMPK inhibitor modulates the activity of orexin neuronal circuits in the hypothalamus.
